# EUROPÄISCHE PATENTSCHRIFT

(11) **EP 2 542 658 B1**
(45) Veröffentlichungstag und Bekanntmachung des Hinweises auf die Patenterteilung: **18.02.2015**
(21) Anmeldenummer: 11705880.0
(22) Anmeldetag: 28.02.2011
(51) Int. Cl.: C12M 1/107

(54) **VERFAHREN UND VORRICHTUNG ZUR ANAEROBEN FERMENTATION**
PROCESS AND DEVICE FOR ANAEROBIC FERMENTATION
PROCÉDÉ ET DISPOSITIF POUR LA FERMENTATION ANAÉROBIE

(30) Priorität: 04.03.2010 DE 102010010294
(43) Veröffentlichungstag der Anmeldung: 09.01.2013
(73) Patentinhaber: Conviotec GmbH, 24941 Flensburg (DE)
(72) Erfinder: SCHNEIDER, Holger, Dr., 24941 Flensburg (DE)
(74) Vertreter: Hansen, Jochen
(86) Internationale Anmeldenummer: PCT/EP2011/052889
(87) Internationale Veröffentlichungsnummer: WO 2011/107419

(56) Entgegenhaltungen:
- EP-A1- 0 335 825
- EP-A1- 0 738 688
- CH-A5- 688 737
- US-B1- 6 660 518

## Beschreibung

Die Erfindung betrifft ein Verfahren zur Erzeugung von Biogas, elektrischer Energie und Wärme aus biologischen Materialien und genauer ein Verfahren zur anaeroben Fermentation eines fließfähigen Substrats unter Verwendung eines Reaktors, mindestens aufweisend:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,

Die Erfindung betrifft im gleichen Maße auch einen Reaktor, wie er für das erfindungsgemäße Verfahren zur Anwendung kommt.

Der Entstehungsprozess von Biogas aus biologischem Ausgangsmaterial unter Ausschluss von Sauerstoff (anaerob) kann im Wesentlichen in vier Schritte unterteilt werden:
▪ Im ersten Schritt, der "Hydrolyse", werden die komplexen Verbindungen des Substratmaterials (z.B. Kohlenhydrate, Eiweiße, Fette) in einfachere organische Verbindungen (z.B. Aminosäuren, Zucker, Fettsäuren) zerlegt. Die daran beteiligten Bakterien setzen hierzu Enzyme frei, die das Material auf biologischem Wege zersetzen.
▪ Im zweiten Schritt werden dann die gebildeten Zwischenprodukte innerhalb der so genannten "Versäuerungsphase" (Acidogenese) durch säurebildende Bakterien weiter zu niederen Fettsäuren (z.B. Essig-, Propion- und Buttersäure) sowie Kohlendioxid (CO2) und Wasserstoff abgebaut. Daneben werden auch geringe Mengen an Milchsäure und Alkoholen gebildet.
▪ Im dritten Schritt werden anschließend die Vorprodukte innerhalb der "Essigsäurebil-dung" (Acetogenese), durch Bakterien zu Vorläufersubstanzen des Biogases (Essigsäure, Wasserstoff und Kohlendioxid) umgesetzt.
▪ In dem letzten Schritt der Biogasherstellung, der "Methanogenese", wird ebenfalls durch Bakterien aus den Produkten der Acetogenese das Methan gebildet.

Laufen die vier Abbauschritte gemeinsam in einem Fermenter ab, spricht man von einstufigen Anlagen. Da die Bakterien der einzelnen Stufen aber unterschiedliche Anforderungen an den Lebensraum stellen, kann eine räumliche Trennung der Abbaustufen von Vorteil sein.

Aus der WO 2006 / 124781 ist eine Biogasanlage für die anaerobe Fermentation bekannt, bei der ein zur Anwendung kommender Reaktor in verschiedene, fest konfektionierte Kammern aufgeteilt ist und in denen die im Absatz [0003] beschriebenen Reaktionen stattfinden.

In der US 2007 / 025 69 71 A1 wird eine transportable Biogasanlage beansprucht, die aus mehreren in ihrer Größe jeweils fest konfektionierten, flexiblen, blasenartigen Kammern besteht.

Aus CH-A5-688737 ist eine Biogasanlage mit einer Mehrzahl von Trennwänden bekannt, die mindestens das für das Substrat bestimmte Innenvolumen des Reaktors in eine Mehrzahl von Kompartimenten unterteilen.

Beiden bekannten Biogasanlagen ist als Nachteil gemeinsam, dass sie in ihrer starren Konfektionierung in einem besonderen Maße auf die Störanfälligkeit der Methanbakterien ausgerichtet sein müssen. Hintergrund dafür ist, dass die Methanbakterien aus der Liste aller an der anaeroben Fermentation planmäßig beteiligten Bakterien am empfindlichsten auf Störungen reagieren und sich überdies nur sehr langsam vermehren. Infolgedessen werden die Milieubedingungen innerhalb der gesamten aus dem Stand der Technik bekannten Reaktoren an die Methanbakterien angepasst.

Der oben genannten räumlichen Trennung der Abbaustufen sind jedoch Grenzen insofern gesetzt, da beispielsweise die Bakterien der Acetogenese auf eine Lebensgemeinschaft mit denen der Methanogenese angewiesen sein können. Der Grund für diese Abhängigkeit liegt darin, dass die Bakterien der Acetogenese einen zu hohen Wasserstoffgehalt nicht vertragen.

Ein besonderes Problem von dem Stand der Technik zuzurechnenden transportablen Anlagen mit kleinen Fermentervolumina ist ihre besonders hohe Störanfälligkeit; Veränderungen der Substratzusammensetzung, welche bei biologischen Materialien naturgemäß gegeben sind und Unregelmäßigkeiten in der Substratzuführung führen hier sehr schnell zu Änderungen des pH-Wertes und zur Verschiebungen der mikrobiellen Besiedelung und damit zur Instabilität des Systems.

Diesen Problemen tritt man zurzeit durch die Verwendung nicht transportabler großtechnischer Biogasanlagen, welche über kontinuierlich durchmischte Reaktoren (CSTR - continuous stirred tank reactor) verfügen, mit bis zu mehreren tausend Kubikme-tern Arbeitsvolumen und langen Verweilzeiten entgegen. Auch diese Systeme zeigen noch eine erhebliche Anfälligkeit gegen Änderungen der Substratzusammensetzung.

In einem kontinuierlich durchmischten Reaktor (CSTR - continuous stirred tank reactor) sind idealerweise an allen Stellen im Reaktor gleiche Fermentations- und Strömungsbedingungen. Dadurch wirken sich Störungen sofort auf den gesamten Reaktorinhalt aus. Dieses Prozessrisiko wird durch sehr niedrige Raumbelastungen (geringe Substratzufuhr pro Zeiteinheit) des Reaktors möglichst gering gehalten.

Der Erfindung liegt daher die Aufgabe zugrunde, der Öffentlichkeit eine Vorrichtung zur Verfügung zu stellen, in der der Biogasprozess bei Veränderung der Substratzusammensetzung und der Substratzuführung stabil bleibt, hohe Raum-Zeit-Ausbeuten ermöglicht, mit kurzen Verweilzeiten effektiv arbeitet, auch in kleinen Fermentervolumina beherrschbar ist und selbst in transportablen Behältern noch mit geringem Wartungs- und Überwachungsaufwand verfahrensstabil abläuft.

Die Aufgabe wird erfindungsgemäß durch ein Verfahren zur anaeroben Fermentation eines fließfähigen Substrats mit definiertem Trockensubstanzgehalt, unter Verwendung eines Reaktors, mindestens aufweisend:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
wobei das hier vorgeschlagene Verfahren gekennzeichnet wird dadurch, dass
□ zur Erhöhung oder Reduktion des Verhältnisses des Volumens der in die eine Richtung vom Substrat durchströmten Kammern (8 (i) - 8 (iv)) zu dem Volumen der in die andere Richtung vom Substrat durchströmten Kammern (9 (i) - 9 (iv)) zumindest ein Teil der Trennwände (6) in ihrer räumlichen Lage und/oder Position und/oder Ausdehnung beweglich angeordnet werden,
□ wobei die Bewegung und/oder Ausdehnung der Trennwände (6) in Abhängigkeit vom Trockensubstanzgehalt des fließfähigen Substrats gesteuert wird.

Bevorzugt ist der für das vorgeschlagene Verfahren zur Anwendung kommende Reaktor ein aus Stahl gefertigter Behälter in kubischer oder zylindrischer Form, bei letztgenannter Bauform mit runder oder elliptischer Form. Zumindest für kleinere Bauformen, wie beispielsweise Versuchsreaktoren, eignet sich als Baumaterial bevorzugt Acrylglas, Kunststoff und Faserverstärkter Kunststoff, für Großreaktoren bieten sich bevorzugt Beton, Stahl und Stahlbeton hinsichtlich des Boden und für die Seitenwände sowie Stahl und Faserverstärkte Kunststoffe für das Dach an, ohne auf die Bauformen und/oder die genannten Baumaterialien im Sinne der vorliegenden Erfindung beschränkt zu sein. Hinsichtlich des Innenvolumens des Reaktors sind Größenordnungen von 4 Liter für Versuchsreaktoren bis 200 m³ für Großreaktoren bevorzugt und vorstellbar.

In einer bevorzugten Ausführungsform des vorgeschlagenen Verfahrens weist der dabei verwendete Reaktor in einer gleichsam bevorzugten Ausführung entlang seiner Längsachse eine Mehrzahl nebeneinander positionierter Kompartimente (7 (i)- 7 (iv)) auf. In zahlreichen der Erfindung vorausgegangenen Versuchen wurden bei dem erfindungsgemäßen Verfahren insbesondere solche Reaktoren verwendet, bei denen jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) - pro Kompartiment die jeweils zuerst vom Substrat durchströmte Kammer - vom Substrat abwärts und die Kammern (9 (i) - 9 (iv)) - pro Kompartiment die jeweils zuletzt vom Substrat durchströmte Kammer - vom Substrat aufwärts durchströmt werden. Derartige Reaktoren gelten gleichsam als bevorzugt im Sinne der Erfindung. Dabei können ganz besonders bevorzugt in Abhängigkeit vom Trockensubstanzgehalt des fließfähigen Substrats zumindest ein Teil der Trennwände (6) entlang der Richtung der Längsachse des Reaktors verschoben werden. Die Unterteilung des Reaktors in eine Mehrzahl von Kompartimenten (7 (i) - 7 (iv)) und die Unterteilung jedes einzelnen Kompartiments (7 (i) - 7 (iv)) jeweils in mindestens zwei Kammern (8 (i) - 8 (iv); 9 (i) - 9 (iv)) geschieht dabei mit einer Mehrzahl von Trennwänden (6), die bevorzugt vertikal ausgerichtet sind.

Die hier vorgeschlagenen Verfahren setzen jedoch keineswegs eine Bauform der zu verwendenden Reaktoren mit entlang der Längsachse des Reaktors nebeneinander positionierten Kompartimenten (7 (i) - 7 (iv)) und mit entlang der Richtung der Längsachse des Reaktors verschiebbaren vertikal ausgerichtet Trennwänden (6) voraus. Genauso vorstellbar sind beispielsweise übereinander liegende Kompartimente (7 (i) - 7 (iv)) mit bevorzugt horizontal ausgerichteten Trennwänden (6).

Hinsichtlich der Trennwände (6) ist neben deren Verschiebung als Bewegungsform auch deren Drehung, Kippen bzw. Klappen, Aus- und Einfahren sowie ein sich räumliches Ausdehnen und sich Verjüngen jeweils in Abhängigkeit vom Trockensubstanzgehalt des Substrats vorstellbar, wobei insbesondere das Drehen, Kippen bzw. Klappen der Trennwände als neben dem Verschieben besonders bevorzugte Ausführungsformen hinsichtlich der Bewegung der Trennwände (6) sind.

Überraschenderweise hat sich gezeigt, dass sich ein Zustand der Autoimmobilisierung der Bakterien in den jeweiligen aufwärts durchströmten Kammern (9 (i) - 9 (iv)) der einzelnen Kompartimente (7 (i) - 7 (iv)) erreichen lässt, wenn der Abstand der Trennwände (6) in erfindungsgemäßer Art und Weise an den Gehalt der Trockensubstanz des verwendeten Substrates angepasst wird. Dabei wird der Trockensubstanzgehalt des Substrats bevorzugt im Zulauf (1) zum Reaktor bemessen. Genauso ist eine Bestimmung des Trockensubstanzgehalts des Substrates im Ablauf (3) möglich, setzt aber dann genauere Kenntnisse zum Prozessverhalten des Reaktors voraus.

Bei der erfindungsgemäßen Anpassung des Abstands der Trennwände (6) an den Gehalt der Trockensubstanz des verwendeten Substrates kommt es so zu einer Entkopplung der hydraulischen von der Feststoffverweilzeit. Durch die gezielte Modifikation der Kammern (8 (i) - 8 (iv); 9 (i) - 9 (iv)) der Kompartimente (7 (i) - 7 (iv)) ergibt sich eine vollkommen neue Methode zur Steuerung des Prozesses der Biogas-Produktion.

Tabelle 1 zeigt für besonders stabil ablaufende Biogas-Herstellungsabläufe das jeweilige und im Sinne der vorliegenden Erfindung besonders bevorzugte Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) in Abhängigkeit vom Trockensubstanzgehaltes des Substrates.

**Tabelle 1**

| Trockensubstanzgehalt in Gew.-% | Verhältnis des jeweiligen Volumens der abwärts vom Substrat durchströmten Kammern (8 (i) - 8 (iv)) zu dem jeweiligen Volumen der aufwärts vom Substrat durchströmten Kammern (9 (i) - 9 (iv)) | Siehe dazu Figur |
|---|---|---|
| < 2 % | 1:3 | 2a |
| 2-5% | 1:2 | 2b |
| 5-10% | 1:1 | 2c |
| 10-15% | 2:1 | 2d |
| 15-20% | 3:1 | 2e |

Der Tabelle lässt sich entnehmen, dass bei dem vorgeschlagenen Verfahren bevorzugt das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) derart eingestellt wird, dass es mit steigendem Trockensubstanzgehalt des Substrates zunimmt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von < 2 Gew.-% in einem Bereich von [1 : 3,5] bis [1 : größer 2,5] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 2 Gew.-% bis 5 Gew.-% in einem Bereich von [1 : 2,5] bis [1 : größer 1,5] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 5 Gew.-% bis 10 Gew.-% in einem Bereich von [1 : 1,5] bis [kleiner 1,5 : 1] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 10 Gew.-% bis 15 Gew.-% in einem Bereich von [größer 1,5 : 1] bis [2,5 : 1] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 15 Gew.-% bis 20 Gew.-% in einem Bereich von [größer 2,5 : 1] bis [3,5 : 1] liegt.

Die Erfindung betrifft im gleichen Maße auch einen Reaktor, wie er für das erfindungsgemäße Verfahren in mindestens einer der bevorzugten Ausführungsformen zur Anwendung kommt. Dabei weist ein solcher Reaktor zur anaeroben Fermentation eines fließfähigen Substrats mit definiertem Trockensubstanzgehalt, mindestens auf:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
wobei der hier vorgeschlagene Reaktor gekennzeichnet wird dadurch, dass
zur Erhöhung oder Reduktion des Verhältnisses des Volumens der in die eine Richtung vom Substrat durchströmten Kammern (8 (i) - 8 (iv)) zu dem Volumen der in die andere Richtung vom Substrat durchströmten Kammern (9 (i) - 9 (iv))
die Trennwände (6) in ihrer räumlichen Lage und/oder Position beweglich angeordnet sind,
und dass der Reaktor mindestens eine Steuerung zur Bewegung der Trennwände (6) in Abhängigkeit vom Trockensubstanzgehalt des fließfähigen Substrats aufweist.

In einer bevorzugten Ausführungsform erstrecken sich die Trennwände (6) über die gesamte Breite des Reaktors.

Die Anordnung der Trennwände (6) erfolgt sinnvollerweise derart, dass die Bildung von Rinnsalen und/oder Pfropfen innerhalb des Reaktors vermieden wird und ein optimaler Fluss durch den Reaktor dauerhaft gewährleistet werden kann.

Die Böden der einzelnen Kompartimente (7 (i) - 7 (iv)) können verschieden gestaltet sein, sie können beispielsweise rund sein oder gerade mit oder ohne Neigung. Sie können auch mit einer oder mehreren Entnahmestellen für das in den Reaktor eingegebene Substrat ausgestattet sein, so dass es möglich ist, Substrat an verschiedenen Stellen aus dem Reaktor zu entnehmen und an anderer Stelle wieder in den Reaktor einzubringen (recyclieren). Zur Entnahme des Substrats kann es vorteilhaft sein, eine Pumpe einzusetzen. Insbesondere eine Monopumpe, die über verschiedene Ventile mit allen intermediären Ablauf- und Zulaufstellen geschaltet ist, kann zu Zwecken des Recyclierens eingesetzt werden.

Der Durchfluss durch den Reaktor wird durch die Anordnung von Zulauf (1) und Ablauf (3) sowie nach dem Prinzip der kommunizierenden Röhren rein hydraulisch vollzogen oder über eine oder mehrere Pumpe unterstützt.

Über den einzelnen Kompartimenten (7 (i) - 7 (iv)) sind Gasräume (5) vorgesehen, die entweder miteinander verbunden sind oder hermetisch getrennt, sodass die Gasentnahme komplett in einem Gasstrom über einen zentralen Gasausgang (2) oder separat in mehreren Gasströmen über mehrere Gasausgänge (2) - beispielsweise pro Kompartiment (7 (i) - 7 (iv)) ein Gasausgang (2) - erfolgen kann.

Der erfindungsgemäße Reaktor kann von seinen Abmaßen so gestaltet werden, dass er in einen Container eingelassen werden kann und somit transportabel ist.

Vor dem Eintrag in den erfindungsgemäßen Reaktor können die zu vergärenden Materialien auf eine geeignet Art und Weise derart vorbehandelt werden, dass die durchschnittliche Partikelgröße ≤ 5mm beträgt.

Der erfindungsgemäße Reaktor/das erfindungsgemäße Verfahren ermöglicht die anaerobe Vergärung bevorzugt von Materialien mit einem Trockenmassegehalt von 2-20% und einem CSB von 3000 mg/l bis 500.000 mg/l.

Der Anteil der nicht vergärbaren Substanzen in dem zugeführten Substrat überschreitet bevorzugt nicht einen Anteil von 20 Gew.-% in der Trockenmasse.

Die in Beschreibung und Patentansprüchen gemachten Angaben zu Gew.-% (Gewichts-%) beziehen sich jeweils auf das "atro"-Gewicht, d.h. absolut trockene Gewichtsteile.

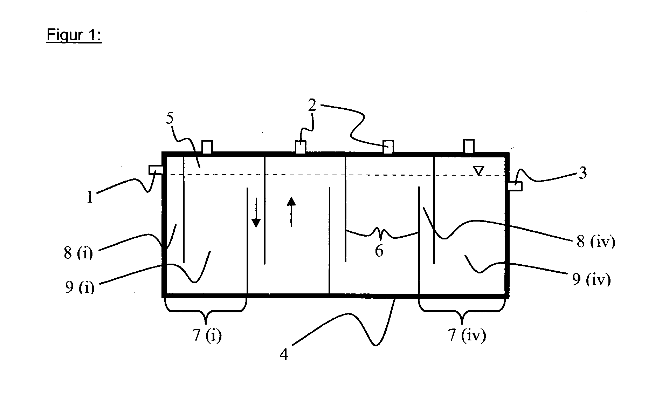

Figur 1stellt eine mögliche Ausführungsform des hier vorgeschlagenen Reaktors zur anaeroben Fermentation eines fließfähigen Substrats mit definiertem Trockensubstanzgehalt dar. Der Reaktor ist dabei ein kubischer Behälter mit horizontal ausgerichtetem rechteckigem Boden (4), vertikalen rechteckigen Kopf- und Seitenwänden und einem horizontal ausgerichtetem Dach. Der Reaktor weist im oberen Bereich der einen Kopfwand eine Zulauf (1) und im oberen Bereich der gegenüberliegenden Kopfwand einen Ablauf (3) auf, durch die dem Reaktor Substrat zugeführt und entnommen werden kann. Über drei - im vorliegenden Fall nicht bewegliche - vertikal vom Boden (4) in den Innenraum des Reaktors hineinragende Trennwände (6) ist der Innenraum des Reaktors in vier Kompartimente (7 (i) - 7 (iv)) unterteilt. Über vier bewegliche - hier entlang der Richtung der Längsachse des Reaktors verschiebbare - Trennwände (6) ist jedes einzelne Kompartiment (7 (i) - 7 (iv)) jeweils in genau zwei gegenläufig vom Substrat durchströmte Kammern (8 (i) - 8 (iv); 9 (i) - 9 (iv)) unterteilt. Dabei werden die vom Substart pro Kompartiment (7 (i) - 7 (iv)) zuerst durchströmten Kammern (8 (i) - 8 (iv)) vom Substrat abwärts gerichtet durchströmt, die vom Substart pro Kompartiment (7 (i) - 7 (iv)) zuletzt durchströmten Kammern (9 (i) - 9 (iv)) vom Substrat aufwärts gerichtet durchströmt. Im oberen Bereich eines jeden Kompartiments (7 (i) - 7 (iv)) ist ein Gasraum (5) ausgebildet mit einem eigenen Gasausgang (2), über den die bei der anaeroben Fermentation entstehenden Gase abgeführt werden können.

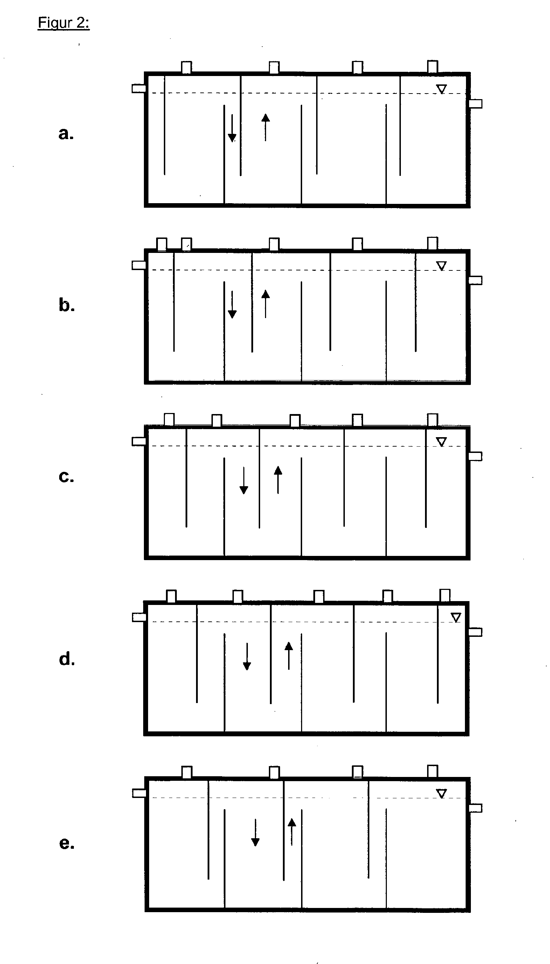

Figur 2 mit ihren Teilfiguren a. bis e. zeigt zur Verdeutlichung die durch Verschiebung der Trennwände (6) beeinflussbaren Verhältnisse des jeweiligen Volumens der abwärts vom Substrat durchströmten Kammern (8 (i) - 8 (iv)) zu dem jeweiligen Volumen der aufwärts vom Substrat durchströmten Kammern (9 (i) - 9 (iv)) für die in der Tabelle 1 dargestellten Fälle.

Zur Demonstration der erfindungsgemäßen Lehre wurden im Folgenden die Allgemeinheit der Lehre nicht einschränkenden Versuche gefahren.

Zum Vergleich wurde ein konventioneller kontinuierlich durchrührter Reaktor (CSTR) mit folgenden Charakteristika verwendet:
Arbeitsvolumen: 4 Liter
Kopfraum: ca. 1 Liter
Rührerdrehzahl: 100 rpm (Umdrehungen pro Minute)

Dieser Laborreaktor ist mit einem Biogasfermenter mit Zentralrührwerk und einem Höhe zu Durchmesser-Verhältnis (H:D) von 1:1 vergleichbar.

Die maximalen Raumbelastungen der geprüften Substrate lagen in diesem Laborreaktor bei:
Maissilagepresssaft: 7 kg oTS/ m³ d
Speisereste: 3 kg oTS/ m³ d (Schaumbildung bei höheren Belastungen)
Zuckerrübe: 7 kg oTS/ m³ d

Bei allen Experimenten mit dem konventionellen Laborreaktor führten Unregelmäßigkeiten in der Substratzufuhr (schwankende Mengen, Qualität oder so genannte "Shock loads" - plötzliche Überfrachtung mit großen Substratmengen) zu einer starken Verminderung der Biogasproduktion bis zum völligen Abbruch des Biogasprozesses.

### Der erfindungsgemäße Versuchsreaktor

Für die Ableitung der erfindungsrelevanten Parameter wurde ein Versuchsreaktor im verkleinerten Maßstab verwendet, der in seinem Grundaufbau der Darstellung in Figur 1 entspricht. Dieser im vorliegenden Fall aus Acrylglas gefertigte Versuchsreaktor hat ein Arbeitsvolumen von 4 Litern mit vier Kompartimenten (7 (i) - 7 (iv)) mit jeweils einer abwärts und einer aufwärts durchströmten Kammer (8 (i) - 8 (iv); 9 (i) - 9 (iv)). Die Kompartimente (7 (i) - 7 (iv)) haben jeweils eine Grundfläche von 0,002 m². Der Innenraum des Reaktors ist 0,5 m hoch mit einem Gasraum (5) von zusätzlich 0,2 m. Die Volumina der Kammern (8 (i) - 8 (iv); 9 (i) - 9 (iv)) sind abhängig vom jeweiligen Verhältnis von abwärts durchströmter Kammer (8 (i) - 8 (iv)) zu aufwärts durchströmter Kammer (8 (i) - 8 (iv)) und erfindungsgemäß dem jeweiligen Trockensubstanzgehalt des Substrats angepasst. Die entsprechenden Strömungsgeschwindigkeiten in den einzelnen Kammern sind aufgeführt. In einem Bioreaktor im Produktionsmaßstab von mehreren Kubikmetern werden die Strömungsgeschwindigkeiten höher liegen (bis 0,5 m/h).

### Analysenmethoden

Der Trockensubstanzgehalt der Substrate (TS) wurde gravimetrisch durch Trocknung einer Probe bei 105°C über 24 h (bis zum konstanten Gewicht) bestimmt und in Prozent der festen Bestandteile angegeben. Die organische Trockensubstanz (oTS) ist der Glühverlust der getrockneten Probe, der bei der Verglühung der Probe bei 600°C entsteht. Die oTS stellt den prozentualen Anteil an organischer Substanz bezogen auf die Trockensubstanz der Probe dar.

Für die Bestimmung der CSB-Werte wurden Küvettentest-Kits (LCK514) der Fa. Hach-Lange verwendet.

Der Biogasertrag wurde mit einem Gaszähler "Milligascounter" der Fa. Ritter bestimmt.

### Aufbereitung der Substrate

Aufgrund der geringen Durchströmgeschwindigkeiten und der damit verbundenen niedrigen Pumpraten der verwendeten Schlauchpumpen war es notwendig, die Substrate besonders vorzubehandeln. Dazu wurden die Substrate in einer Laborhammermühle mit einem Siebdurchmesser von 0,5mm vermahlen und homogenisiert.

### Ergebnisse der Testfermentationen

Einzelne Testsubstrate wurden im Versuchreaktor bei 37°C und unter Zusatz einer Impfkultur, die aus entgastem Klärschlamm einer Anaerobstufe einer kommunalen Kläranlage stammte, vergoren.

### Substrate mit einem Trockensubstanzgehalt von 2 - 5 % (Behandlung mittels erfindungsgemäßem Reaktor und Anwendung des erfindungsgemäßen Verfahrens)

Testsubstrat: Schweinegülle mit 4 % Trockensubstanzgehalt
Verweilzeit: 12 Tage
Verhältnis der abwärts- zur aufwärtsdurchströmten Kammer: 1:2
Strömungsgeschwindigkeit in der abwärtsdurchströmten Kammer: 0,02 m/h
Strömungsgeschwindigkeit in der aufwärtsdurchströmten Kammer: 0,01 m/h
CSB am Reaktoreinlauf: 40.000 - 50.000 mg/l
CSB am Reaktorauslauf: 2.000 - 3.000 mg/l
Raumbelastung: max. 3 kg oTS/m³ d
Biogasertrag: 20m³ je t Schweinegülle

### Substrate mit einem Trockensubstanzgehalt von 5 - 10 % (Behandlung mittels erfindungsgemäßem Reaktor und Anwendung des erfindungsgemäßen Verfahrens)

Testsubstrat: thermisch vorbehandelter Maissilagepresssaft mit 9 % Trockensubstanzgehalt
Verweilzeit: 8 Tage
Verhältnis der abwärts- zur aufwärtsdurchströmten Kammer: 1:1
Strömungsgeschwindigkeit in der abwärtsdurchströmten Kammer: 0,02 m/h
Strömungsgeschwindigkeit in der aufwärtsdurchströmten Kammer: 0,02 m/h
CSB am Reaktoreinlauf: 120.000 - 140.000 mg/l
CSB am Reaktorauslauf: 500 - 1.000 mg/l
Raumbelastung: max. 11 kg oTS/m³ d
Biogasertrag: 65m³ je t Maissilagepresssaft

### Substrate mit einem Trockensubstanzgehalt von 10 - 15 % (Behandlung mittels erfindungsgemäßem Reaktor und Anwendung des erfindungsgemäßen Verfahrens)

Testsubstrat: hygienisierte Speisereste mit 14,5 % Trockensubstanzgehalt
Verweilzeit: 10 Tage
Verhältnis der abwärts- zur aufwärtsdurchströmten Kammer: 2:1
Strömungsgeschwindigkeit in der abwärtsdurchströmten Kammer: 0,012 m/h
Strömungsgeschwindigkeit in der aufwärtsdurchströmten Kammer: 0,025 m/h
CSB am Reaktoreinlauf: 200.000 - 230.000 mg/l
CSB am Reaktorauslauf: 1.000 - 2.000 mg/l
Raumbelastung: max. 15 kg oTS/m³ d
Biogasertrag: 131m³ je t Speisereste

### Substrate mit einem Trockensubstanzgehalt von 15 - 20 % (Behandlung mittels erfindungsgemäßem Reaktor und Anwendung des erfindungsgemäßen Verfahrens)

Testsubstrat: zerkleinerte Zuckerrüben mit 19 % Trockensubstanzgehalt
Verweilzeit: 10 Tage
Verhältnis der abwärts- zur aufwärtsdurchströmten Kammer: 3:1
Strömungsgeschwindigkeit in der abwärtsdurchströmten Kammer: 0,01 m/h
Strömungsgeschwindigkeit in der aufwärtsdurchströmten Kammer: 0,033 m/h
CSB am Reaktoreinlauf: 270.000 - 300.000 mg/l
CSB am Reaktorauslauf: 1.000 - 2.000 mg/l
Raumbelastung: max. 20 kg oTS/m³ d
Biogasertrag: 153m³ je t Zuckerrüben

Beim erfindungsgemäßen Verfahren zeigt sich in allen Fällen eine Unempfindlichkeit gegenüber Substratschwankungen, sowohl in Bezug auf die Menge wie auch in Bezug auf die Qualität des Substrates.

Der erfindungsgemäße Reaktor ist durch die Kompartimentierung robust gegen Schwankungen und Störungen. Dadurch sind im erfindungsgemäßen Reaktor höhere Raumbelastungen möglich. In den einzelnen Kompartimenten stellen sich unterschiedliche Fermentationsbedingungen (wie z.B. pH-Werte) ein, die zur Stabilisierung des Fermentationsprozesses führen. Der Biogasprozess besteht aus verschiedenen Einzelschritten, die zwar aufeinander aufbauen aber unter unterschiedlichen Bedingungen optimal ablaufen. Der erfindungsgemäße Reaktor unterstützt speziell diese Prozessbesonderheiten. Die höheren Raumbelastungen, kürzeren Verweilzeiten und die besseren Fermentationsbedingungen führen zu insgesamt höheren Erträgen pro Zeiteinheit (erhöhte Raum-Zeit-Ausbeute) verglichen mit konventionellen Reaktoren, wie der nachfolgenden Tabelle 2 zu entnehmen ist.

**Tabelle 2: Erhöhung der Raum-Zeit-Ausbeute des erfindungsgemäßen Laborreaktors im Vergleich zum konventionellen Laborreaktor:**

| Testsubstrat | Raum-Zeit-Ausbeute [m³ Biogas je m³ Reaktorvolumen und Tag] | | Erhöhung der Raum-Zeit-Ausbeute |
|---|---|---|---|
| | erfindungsgemäßer Laborreaktor | konventioneller Laborreaktor | |
| Schweinegülle | 2 | 0,7 | 3 x |
| vorbehandelter Maissilagepresssaft | 9 | 3 | 3 x |
| hygienisierte Speisereste | 14 | 2 | 7 x |
| zerkleinerte Zuckerrüben | 16 | 4 | 4 x |

### Bezugszeichenliste:

- 1: Zulauf
- 2: Gasausgang
- 3: Ablauf
- 4: Boden
- 5: Gasraum
- 6: Trennwände
- 7 (i) bis 7 (iv): Kompartimente (i) bis (iv)
- 8 (i) bis 8 (iv): abwärts durchströmte Kammern in den Kompartimenten (i) bis (iv)
- 9 (i) bis 9 (iv): aufwärts durchströmte Kammern in den Kompartimenten (i) bis (iv)

## Patentansprüche

1. Verfahren zur anaeroben Fermentation eines fließfähigen Substrats mit definiertem Trockensubstanzgehalt, unter Verwendung eines Reaktors, mindestens aufweisend:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
**dadurch gekennzeichnet, dass**
zur Erhöhung oder Reduktion des Verhältnisses des Volumens der in die eine Richtung vom Substrat durchströmten Kammern (8 (i) - 8 (iv)) zu dem Volumen der in die andere Richtung vom Substrat durchströmten Kammern (9 (i) - 9 (iv)) zumindest ein Teil der Trennwände (6) in ihrer räumlichen Lage und/oder Position und/oder Ausdehnung beweglich angeordnet werden,
wobei die Bewegung und/oder Ausdehnung der Trennwände (6) in Abhängigkeit vom Trockensubstanzgehalt des fließfähigen Substrats gesteuert wird.

2. Verfahren nach Patentanspruch 1, **dadurch gekennzeichnet, dass** der Reaktor entlang seiner Längsachse eine Mehrzahl nebeneinander positionierter Kompartimente (7 (i) - 7 (iv)) aufweist.

3. Verfahren nach Patentanspruch 2, **dadurch gekennzeichnet, dass** jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) vom Substrat abwärts und die Kammern (9 (i) - 9 (iv)) vom Substrat aufwärts durchströmt werden.

4. Verfahren nach einem der Patentansprüche 2 oder 3, **dadurch gekennzeichnet, dass** in Abhängigkeit vom Trockensubstanzgehalt des fließfähigen Substrats zumindest ein Teil der Trennwände (6) zu ihrer Ausrichtung entlang der Richtung der Längsachse des Reaktors verschiebbar angeordnet werden.

5. Verfahren nach einem der Patentansprüche 3 oder 4, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) mit steigendem Trockensubstanzgehalt des Substrates zunimmt.

6. Verfahren nach einem der Patentansprüche 3 bis 5, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) bei einem Trockensubstanzgehalt von < 2 Gew.-% in einem Bereich von [1 : 3,5] bis [1 : größer 2,5] liegt.

7. Verfahren nach einem der Patentansprüche 3 bis 5, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) bei einem Trockensubstanzgehalt von 2 Gew.-% bis 5 Gew.-% in einem Bereich von [1 : 2,5] bis [1 : größer 1,5] liegt.

8. Verfahren nach einem der Patentansprüche 3 bis 5, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) bei einem Trockensubstanzgehalt von 5 Gew.-% bis 10 Gew.-% in einem Bereich von [1 : 1,5] bis [kleiner 1,5 : 1] liegt.

9. Verfahren nach einem der Patentansprüche 3 bis 5, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) bei einem Trockensubstanzgehalt von 10 Gew.-% bis 15 Gew.-% in einem Bereich von [größer 1,5 : 1] bis [2,5 : 1] liegt.

10. Verfahren nach einem der Patentansprüche 3 bis 5, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) bei einem Trockensubstanzgehalt von 15 Gew.-% bis 20 Gew.-% in einem Bereich von.[größer2,5 : 1] bis [3,5 : 1] liegt.

11. Reaktor zur anaeroben Fermentation eines fließfähigen Substrats mit definiertem Trockensubstanzgehalt, mindestens aufweisend:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
**dadurch gekennzeichnet, dass**
zur Erhöhung oder Reduktion des Verhältnisses des Volumens der in die eine Richtung vom Substrat durchströmten Kammern (8 (i) - 8 (iv) zu dem Volumen der in die andere Richtung vom Substrat durchströmten Kammern (9 (i) - 9 (iv) zumindest ein Teil der Trennwände (6) in ihrer räumlichen Lage und/oder Position beweglich angeordnet sind,
und dass der Reaktor mindestens eine Steuerung zur Bewegung der Trennwände (6) in Abhängigkeit vom Trockensubstanzgehalt des fließfähigen Substrats aufweist.

12. Reaktor nach Patentanspruch 11, **dadurch gekennzeichnet, dass** der Reaktor entlang seiner Längsachse eine Mehrzahl nebeneinander positionierter Kompartimente (7 (i) - 7 (iv)) aufweist.

13. Reaktor nach Patentanspruch 12, **dadurch gekennzeichnet, dass** jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) vom Substrat abwärts und die Kammern (9 (i) - 9 (iv)) vom Substrat aufwärts durchströmt sind.

14. Reaktor nach einem der Patentansprüche 12 oder 13, **dadurch gekennzeichnet, dass** zumindest ein Teil der Trennwände (6) in Richtung der Längsachse des Reaktors verschiebbar angeordnet sind.

15. Reaktor nach einem der Patentansprüche 11 bis 14, **dadurch gekennzeichnet, dass** sich die Trennwände (6) über die gesamte Breite des Reaktors erstrecken.

## Claims

1. A process for the anaerobic fermentation of a flowable substrate with defined dry solids content, using a reactor, having at least:
• an inlet (1),
• an outlet (3),
• a plurality of separating walls (6) which divide at least the reactor internal-volume intended for the substrate into a plurality of compartments (7 (i) - 7 (iv)) and each individual compartment (7 (i) - 7 (iv)) in each case into at least two chambers (8 (i) - 8 (iv); 9 (i) - 9 (iv)) flowed through in opposite directions by the substrate,
**characterised in that**
to increase or reduce the ratio of the volume of the chambers (8 (i) - 8 (iv)) flowed through in one direction by the substrate to the volume of the chambers (9 (i) - 9 (iv)) flowed through in the opposite direction by the substrate
at least some of the separating walls (6) are movably arranged in their spatial location and/or position and/or spread,
wherein the motion and/or spread of the separating walls (6) is/are controlled dependent on the dry solids content of the flowable substrate.

2. A process according to claim 1, **characterised in that** the reactor has along its longitudinal axis a plurality of compartments (7 (i) - 7 (iv)) positioned side by side.

3. A process according to claim 2, **characterised in that** each individual compartment (7 (i) - 7 (iv)) is in each case divided into two chambers (8 (i) - 8 (iv); 9 (i) - 9 (iv)) flowed through in opposite directions by the substrate, of which the chambers (8 (i) - 8 (iv)) are downwardly flowed through by the substrate and the chambers (9)i) - 9 (iv)) are upwardly flowed through by the substrate.

4. A process according to one of claims 2 and 3, **characterised in that** dependent on the dry solids content of the flowable substrate, at least some of the separating walls (6) are displaceably arranged with respect to their orientation along the direction of the longitudinal axis of the reactor.

5. A process according to one of claims 3 and 4, **characterised in that** the ratio of the respective volume of the chambers (8 (i) - 8 (iv) downwardly flowed through by the substrate to the volume of the chambers (9 (i) - 9 (iv) upwardly flowed through by the substrate increases as the dry solids content of the substrate rises.

6. A process according to any one of claims 3 to 5, **characterised in that** the ratio of the respective volume of the chambers (8 (i) - 8 (iv) downwardly flowed through by the substrate to the volume of the chambers (9 (i) - 9 (iv) upwardly flowed through by the substrate lies in a range from [1 : 3.5] to [1 : greater than 2.5] in the case of a dry solids content of < 2 wt%.

7. A process according to any one of claims 3 to 5, **characterised in that** the ratio of the respective volume of the chambers (8 (i) - 8 (iv) downwardly flowed through by the substrate to the volume of the chambers (9 (i) - 9 (iv) upwardly flowed through by the substrate lies in a range from [1 : 2.5] to [1 : greater than 1.5] in the case of a dry solids content from 2 wt% to 5 wt%.

8. A process according to any one of claims 3 to 5, **characterised in that** the ratio of the respective volume of the chambers (8 (i) - 8 (iv) downwardly flowed through by the substrate to the volume of the chambers (9 (i) - 9 (iv) upwardly flowed through by the substrate lies in a range from [1 : 1.5] to [less than 1.5 : 1] in the case of a dry solids content from 5 wt% to 10 wt%.

9. A process according to any one of claims 3 to 5, **characterised in that** the ratio of the respective volume of the chambers (8 (i) - 8 (iv) downwardly flowed through by the substrate to the volume of the chambers (9 (i) - 9 (iv) upwardly flowed through by the substrate lies in a range from [greater than 1.5 : 1] to [2.5 : 1] in the case of a dry solids content from 10 wt% to 15 wt%.

10. A process according to any one of claims 3 to 5, **characterised in that** the ratio of the respective volume of the chambers (8 (i) - 8(iv) downwardly flowed through by the substrate to the volume of the chambers (9 (i) - 9 (iv) upwardly flowed through by the substrate lies in a range from [greater than 2.5 : 1] to [3.5 : 1] in the case of a dry solids content from 15 wt% to 20 wt%.

11. A reactor for the anaerobic fermentation of a flowable substrate with defined dry solids content, at least having:
• an inlet (1),
• an outlet (3),
• a plurality of separating walls (6) which divide at least the reactor internal-volume intended for the substrate into a plurality of compartments (7 (i) - 7 (iv)) and each individual compartment (7 (i) - 7 (iv)) in each case into at least two chambers (8 (i) - 8 (iv); 9 (i) - 9 (iv)) flowed through in opposite directions by the substrate,
**characterised in that**
to increase or reduce the ratio of the volume of the chambers (8 (i) - 8 (iv) flowed through in one direction by the substrate to the volume of the chambers (9 (i) - 9 (iv) flowed through in the opposite direction by the substrate
at least some of the separating walls (6) are movably arranged in their spatial location and/or position,
and **in that** the reactor has at least one control means for motion of the separating walls (6) dependent on the dry solids content of the flowable substrate.

12. A reactor according to claim 11, **characterised in that** the reactor has along its longitudinal axis a plurality of compartments (7 (i) - 7 (iv)) positioned side by side.

13. A reactor according to claim 12, **characterised in that** each individual compartment (7 (i) - 7 (iv)) is in each case divided into two chambers (8 (i) - 8 (iv); 9 (i) - 9 (iv)) flowed through in opposite directions by the substrate, of which the chambers (8 (i) - 8 (iv)) are downwardly flowed through by the substrate and the chambers (9 (i) - 9 (iv)) are upwardly flowed through by the substrate.

14. A reactor according to one of claims 12 and 13, **characterised in that** at least some of the separating walls (6) are displaceably arranged in the direction of the longitudinal axis of the reactor.

15. A reactor according to any one of claims 11 to 14, **characterised in that** the separating walls (6) extend over the entire width of the reactor.

## Revendications

1. Procédé de fermentation anaérobie d'un substrat apte à s'écouler ayant une teneur en substances sèches définie, en utilisant un réacteur, comportant au moins :
- une alimentation (1),
- une évacuation (3),
- une pluralité de parois de séparation (6) qui scindent au moins le volume intérieur du réacteur destiné au substrat en une pluralité de compartiments (7 (i) - 7 (iv)) et chacun des compartiments (7 (i) - 7 (iv)) en au moins deux chambres (8 (i) - 8 (iv) ; 9 (i) - (iv)) traversées par le substrat dans des sens opposés,
**caractérisé en ce que**
pour augmenter ou réduire le rapport entre le volume des chambres (8 (i) - 8(iv)) traversées dans un sens par le substrat et le volume des chambres (9 (i) - 9 (iv)) traversées dans l'autre sens par le substrat, au moins une partie des parois de séparation (6) sont disposées de manière mobile du point de vue de leur situation spatiale et/ou leur position et/ou leur dimension,
le déplacement et/ou la dimension des parois de séparation (9) étant commandés en fonction de la teneur en substances sèches dans le substrat apte à s'écouler.

2. Procédé selon la revendication 1, **caractérisé en ce que** le réacteur présente le long de son axe longitudinal une pluralité de compartiments (7 (i) - 7 (iv)) positionnés les uns à côté des autres.

3. Procédé selon la revendication 2, **caractérisé en ce que** chacun des compartiments (7 (i) - 7 (iv)) est scindé en deux chambres (8 (i) - 8 (iv) ; 9 (i) - 9 (iv)) traversées par le substrat dans des sens opposés, les chambres (8 (i) - 8 (iv)) étant traversées vers le bas par le substrat et les chambres (9 (i) - 9 (iv)) étant traversées vers le haut par le substrat.

4. Procédé selon l'une des revendications 2 ou 3, **caractérisé en ce qu'**en fonction de la teneur en substances sèches du substrat apte à s'écouler, au moins une partie des parois de séparation (6) sont disposées mobiles le long de la direction de l'axe longitudinal du réacteur pour leur orientation.

5. Procédé selon l'une des revendications 3 ou 4, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat augmente avec l'augmentation de la teneur en substances sèches dans le substrat.

6. Procédé selon l'une des revendications 3 à 5, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat se situe pour une teneur en substances sèches < 2 % en poids dans une fourchette comprise entre [1 : 3,5] et [1 : supérieur à 2,5].

7. Procédé selon l'une des revendications 3 à 5, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat se situe pour une teneur en substance sèche comprise entre 2 % en poids et 5 % en poids dans une fourchette comprise entre [1 : 2,5] et [1 : supérieur à 1,5].

8. Procédé selon l'une des revendications 3 à 5, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat se situe pour une teneur en substance sèche comprise entre 5 % en poids et 10 % en poids dans une fourchette comprise entre [1 : 1,5] et [inférieur à 1,5 : 1].

9. Procédé selon l'une des revendications 3 à 5, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat se situe pour une teneur en substance sèche comprise entre 10 % en poids et 15 % en poids dans une fourchette comprise entre [supérieur à 1,5 : 1] et [2,5 : 1]

10. Procédé selon l'une des revendications 3 à 5, **caractérisé en ce que** le rapport entre de chacun des volumes des chambres (8 (i) - 8 (iv)) traversées vers le bas par le substrat et le volume des chambres (9 (i) - 9 (iv)) traversées vers le haut par le substrat se situe pour une teneur en substance sèche comprise entre 15 % en poids et 20 % en poids dans une fourchette comprise entre [supérieur à 2,5 : 1] et [3,5 : 1].

11. Réacteur pour la fermentation anaérobie d'un substrat apte à s'écouler ayant une teneur en substances sèches définie, comprenant au moins :
- une alimentation (1),
- une évacuation (3),
- une pluralité de parois de séparation (6) qui scindent au moins le volume intérieur du réacteur destiné au substrat en une pluralité de compartiments (7 (i) - 7 (iv)) et chacun des compartiments (7 (i) - 7 (iv)) en au moins deux chambres (8 (i) - 8 (iv) ; 9 (i) - (iv)) traversées par le substrat dans des sens opposés,
**caractérisé en ce que**
pour augmenter ou réduire le rapport entre le volume des chambres (8 (i) - 8(iv)) traversées dans un sens par le substrat et le volume des chambres (9 (i) - 9 (iv)) traversées dans l'autre sens par le substrat, au moins une partie des parois de séparation (6) sont disposées mobiles du point de vue de leur situation spatiale et/ou leur position et **en ce que** le réacteur comprend au moins une commande pour déplacer les parois de séparation (6) en fonction de la teneur en substances sèches dans le substrat apte à s'écouler.

12. Réacteur selon la revendication 11, **caractérisé en ce que** Procédé selon la revendication 1, **caractérisé en ce que** le réacteur présente le long de son axe longitudinal une pluralité de compartiments (7 (i) - 7 (iv)) positionnés les uns à côté des autres.

13. Réacteur selon la revendication 12, **caractérisé en ce que** chacun des compartiments (7 (i) - 7 (iv)) est scindé en deux chambres (8 (i) - 8 (iv) ; 9 (i) - 9 (iv)) traversées par le substrat dans des sens opposés, les chambres (8 (i) - 8 (iv)) étant traversées vers le bas par le substrat et les chambres (9 (i) - 9 (iv)) étant traversées vers le haut par le substrat.

14. Réacteur selon l'une des revendications 12 ou 13, **caractérisé en ce qu'**au moins une partie des parois de séparation (6) sont disposées mobiles en direction de l'axe longitudinal du réacteur.

15. Réacteur selon l'une des revendications 11 à 14, **caractérisé en ce que** les parois de séparation (6) s'étendent sur toute la largeur du réacteur.
